# EUROPEAN PATENT SPECIFICATION

(11) **EP 3 462 931 B1**
(45) Date of publication and mention of the grant of the patent: **23.06.2021**
(21) Application number: 17724091.8
(22) Date of filing: 22.05.2017
(51) Int. Cl.: A24F 47/00, H05B 3/34

(54) **FLUID PERMEABLE HEATER ASSEMBLY FOR AEROSOL-GENERATING SYSTEMS AND FLAT ELECTRICALLY CONDUCTIVE FILAMENT ARRANGEMENT FOR FLUID PERMEABLE HEATER ASSEMBLIES**
FLUIDDURCHLÄSSIGE HEIZUNGSANORDNUNG FÜR AEROSOLERZEUGUNGSSYSTEME UND FLACHE, ELEKTRISCH LEITENDE FILAMENTANORDNUNG FÜR FLUIDDURCHLÄSSIGE HEIZUNGSANORDNUNGEN
ENSEMBLE DE RADIATEUR POUR PERMÉABLE AUX FLUIDES ET SYSTÈMES DE GÉNÉRATION D'AÉROSOL ÉLECTRIQUEMENT CONDUCTRICE PLATE PERMÉABLE AUX FLUIDES, AGENCEMENT DE FILAMENTS POUR ENSEMBLES DE CHAUFFAGE

(30) Priority: 31.05.2016 EP 16172195
(43) Date of publication of application: 10.04.2019
(73) Proprietor: Philip Morris Products S.A., 2000 Neuchâtel (CH)
(72) Inventor: MIRONOV, Oleg, 1588 Cudrefin (CH); ZINOVIK, Ihar Nikolaevich, 2034 Peseux (CH)
(74) Representative: Spencer, James Michael
(86) International application number: PCT/EP2017/062251
(87) International publication number: WO 2017/207320

(56) References cited:
- WO-A1-2015/117701
- CN-B- 102 425 023
- CN-B- 102 443 954
- DE-U1- 20 111 067

## Description

The invention relates to fluid permeable heater assemblies for aerosol-generating systems and electrically conductive flat filament arrangements for such fluid permeable heater assemblies.

International patent application publication number WO 2015/117701 A1, in the name of Philip Morris Products S.A., describes a fluid permeable heater assembly for aerosol-generating systems comprising a substrate comprising an opening through the substrate, an electrically conductive substantially flat filament arrangement arranged over the opening, and clamping means mechanically fixing the filament arrangement to the substrate. The clamping means are electrically conductive and serve as electrical contacts for providing a heating current through the filament arrangement.

It would be desirable to have fluid permeable heaters and filament arrangements for such fluid permeable heaters that may improve the performance of the corresponding heaters. In particular, it would be desirable to have a heater assembly and a filament arrangement having optimized contact and heating performance.

According to an aspect of the invention, there is provided an electrically conductive flat filament arrangement for a fluid permeable heater assembly for aerosol-generating systems, preferably for a heater assembly according to the invention and as defined herein. The flat filament arrangement comprises a center portion and two side portions, wherein the two side portions are arranged on opposite sides of the center portion. The center portion defines a heating region of the filament arrangement and the side portions define electrical contact regions of the filament arrangement. The center portion and the two side portions each comprise a plurality of openings, each plurality of openings defining an open area of the center portion and an open area of each of the two side portions. The percentage of the total area of the center portion comprising the open area of the center portion is greater than the percentage of the total area of one of the side portions comprising the open area of the side portion.

The percentage of the total area of the center portion comprising the open area of the center portion may be greater than the percentage of the total area of each of the side portions comprising the open area of the side portion. The percentage of the total area of the center portion comprising the open area of the center portion may be greater than the percentage of the total area of both of the side portions comprising the open area of the side portions.

A ratio of the percentage of the total area of the center portion comprising the open area of the center portion to the percentage of the total area of one of the two side portions comprising the open area of the side portion may be between 1.1 and 30. The ratio of the percentage of the total area of the center portion comprising the open area of the center portion to the percentage of the total area of one of the two side portions comprising the open area of the side portion may be between 2 and 28, for example between 2 and 15 or between 15 and 28.

According to another aspect of the invention, there is provided an electrically conductive flat filament arrangement for a fluid permeable heater assembly for aerosol-generating systems, preferably for a heater assembly according to the invention and as defined herein. The flat filament arrangement comprises a center portion and two side portions, wherein the two side portions are arranged on opposite sides of the center portion. The center portion defines a heating region of the filament arrangement and the side portions define electrical contact regions of the filament arrangement. The center portion and the two side portions each comprise a plurality of openings, each plurality of openings defining an open area of the center portion and an open area of each of the two side portions. A ratio of the open area in the center portion to the open area of one of the two side portions is between 1.1 and 30. Preferably, the ratio of the open area of the center portion to the open area of one of the two side portions are between 2 and 28, for example between 2 and 15 or between 15 and 28.

The open area in the center portion may be between about 40 percent and about 90 percent of the total area of the center portion. Preferably, the open area in the center portion is between about 50 percent and about 80 percent, more preferably between about 50 and about 70 percent.

The open area in each of the two side portions is larger than zero and may be larger than about 3 percent and smaller than about 40 percent of each of the total areas of the two side portions. Preferably, the open area in each of the side portions are larger than about 5 percent and smaller than about 35 percent, more preferably smaller than about 20 percent, for example the open area in each of the two side portions is between about 5 percent and about 15 percent.

A center portion of the filament arrangement is defined to extend over or comprise about 20 percent of the size of the filament arrangement but may extend to up to about 60 percent of the size of the filament arrangement. A side portion is defined to extend over or comprise about 20 percent of the size of the filament arrangement but may extend to up to about 40 percent of the size of the filament arrangement. Thus, a center portion corresponding to a heating region of the filament arrangement having a large open area, for example of about 65 percent to about 85 percent, may be reduced to about 20 percent of the size of the filament arrangement. The remaining size of the filament arrangement may be side portions having little open area, for example each between about 5 percent and about 10 percent, and where no or little heating occurs. The remaining size of the filament arrangement may also be side portions and transitions portions arranged between side portions and center portion as will be described in more detail below.

According to another aspect of the invention, there is provided a fluid permeable heater assembly for aerosol-generating systems. The fluid permeable heater assembly comprises an electrically conductive flat filament arrangement, and a first contact point and a second contact point for electrically contacting the flat filament arrangement. A longitudinal axis is defined between the first contact point and the second contact point. In the heater assembly, a center surface Sc is an area of the heater assembly extending between two lines lying perpendicular to the longitudinal axis and crossing the longitudinal axis at two points arranged on the longitudinal axis, one of the two points being situated at a distance from the first contact point equal to 40 percent and the other one of the two points being situated at a distance from the first contact point equal to 60 percent of the distance between the first and the second contact point. A first side surface S1 is an area of the heater assembly extending between two lines lying perpendicular to the longitudinal axis and crossing the longitudinal axis at the first contact point and a point arranged on the longitudinal axis and situated at a distance from the first contact point equal to 20 percent of the distance between the first and the second contact point. A second side surface S2 is an area of the heater assembly between two lines lying perpendicular to the longitudinal axis and crossing the longitudinal axis at the second contact point and a point arranged on the longitudinal axis and situated at a distance from the first contact point equal to 80 percent of the distance between the first and the second contact point.

The center surface Sc comprises a plurality of openings defining an open area ScOA, the first side surface S1 comprises a plurality of openings defining an open area S1OA, and the second side surface S2 comprises a plurality of openings defining an open area S2OA.

The percentage of the total area of the center surface comprising the open area of the center surface is greater than the percentage of the total area of the first side surface comprising the open area of the first side surface and the percentage of the total area of the center surface comprising the open area of the center surface is greater than the percentage of the total area of the second side surface comprising the open area of the second side.

A ratio of the percentage of the total area of the center surface comprising the open area of the center surface to the percentage of the total area of the center surface comprising the open area of the first side surface ScOA/S1OA may be between 1.1 and 30, and a ratio of the percentage of the total area of the center surface comprising the open area of the center surface to the percentage of the total area of the second side surface comprising the open area of the second side surface ScOA/S2OA may be between 1.1 and 30.

According to another aspect of the invention, there is provided a fluid permeable heater assembly for aerosol-generating systems. The fluid permeable heater assembly comprises an electrically conductive flat filament arrangement, and a first contact point and a second contact point for electrically contacting the flat filament arrangement. A longitudinal axis is defined between the first contact point and the second contact point. In the heater assembly, a center surface Sc is an area of the heater assembly extending between two lines lying perpendicular to the longitudinal axis and crossing the longitudinal axis at two points arranged on the longitudinal axis, one of the two points being situated at a distance from the first contact point equal to 40 percent and the other one of the two points being situated at a distance from the first contact point equal to 60 percent of the distance between the first and the second contact point. A first side surface S1 is an area of the heater assembly extending between two lines lying perpendicular to the longitudinal axis and crossing the longitudinal axis at the first contact point and a point arranged on the longitudinal axis and situated at a distance from the first contact point equal to 20 percent of the distance between the first and the second contact point. A second side surface S2 is an area of the heater assembly between two lines lying perpendicular to the longitudinal axis and crossing the longitudinal axis at the second contact point and a point arranged on the longitudinal axis and situated at a distance from the first contact point equal to 80 percent of the distance between the first and the second contact point.

The center surface Sc comprises a plurality of openings defining an open area ScOA, the first side surface S1 comprises a plurality of openings defining an open area S1OA, and the second side surface S2 comprises a plurality of openings defining an open area S2OA. A ratio of the open area of the center surface to the open area of the first side surface ScOA/S1OA is between 1.1 and 30, and a ratio of the open area of the center surface to the open area of the second side surface ScOA/S2OA is between 1.1 and 30. Preferably, the ratio of the open area of the center surface to the first side surface or to the second side surface, ScOA/S1OA, or ScOA/S2OA are between 2 and 28, for example between 2 and 15 or between 15 and 28.

The open area of the center surface ScOA may be between about 40 percent and about 90 percent of the total area of the center surface. Preferably, the open area in the center surface is between about 50 percent and about 80 percent, more preferably between about 50 and about 70 percent.

A heater assembly may have a constant width along the length of the longitudinal axis with respect to the filament arrangement. A heater assembly may have a varying width along the length of the longitudinal axis. In these cases, for the purpose of calculating the open areas, the heater assembly is considered to be the rectangular area between two lines parallel to the longitudinal axis passing through points of the filament arrangement which are the most distant to the longitudinal axis. By this, the absence of filament arrangement in narrower parts of the heater assembly is counted as open area.

As a general rule, whenever the term "about" is used in connection with a particular value throughout this application this is to be understood such that the value following the term "about" does not have to be exactly the particular value due to technical considerations. However, the term "about" used in connection with a particular value is always to be understood to include and also to explicitly disclose the particular value following the term "about"

The open area in each of the two side surfaces S1OA, S2OA is larger than zero and may be larger than about 3 percent and smaller than about 40 percent of each of the total areas of the two side surfaces. Preferably, the open area in each of the side surfaces are larger than about 5 percent and smaller than about 35 percent, more preferably smaller than about 20 percent, for example the open area in each of the two side surfaces is between about 5 percent and about 15 percent.

Most of the heating may happen in a central surface of the heater assembly between the two contact points. Little heating may happen in the side surfaces.

This variability in sizes of a heating region and side or contact regions allow to vary, in particular enlarge, a size of a heater assembly, in particular a filament arrangement of the heater assembly, however, without varying too much, in particular enlarging a heating region. This may be required or desired in order to not impose excessive demands to a power system of an aerosol-generating device.

The open area of a center surface is provided and may be required for a liquid to pass into the center surface of the heater assembly, be heated and evaporated by the heated filament arrangement.

The open area of the center surface is formed by a plurality of openings. The plurality of openings preferably has a size and distribution optimized for a fluid to penetrate into the openings and allow a direct and efficient heating of the fluid.

It has been found that an open area in the form of a plurality of openings in side surfaces may be beneficial to the performance of a heater assembly. The open area of each side surface is smaller than the open area of the center surface. However, the open areas of the side surfaces may also have a minimum value of, for example, about 3 percent, and a value of smaller than about 40 percent of the total area of the side surface.

Providing an open area ratio between the open area of the center surface and the open area of the side surface in the above defined ranges has been found to optimize the performance of a heater assembly in view of resistive heating and contacting a filament arrangement in the heater assembly.

Small or little open area in side surfaces, which side surfaces are used as contact portions of the filament arrangement in the heater, may positively influence an electrical contact of these side surfaces compared to, for example, meshes having low densities, for example, like meshes preferred for center portions of a filament arrangement.

In addition, a plurality of openings in side surfaces may limit leakage of liquid out of a heater assembly. Typically, liquid is supplied from a liquid storage reservoir, for example a tank system or cartridge to the heater assembly. The liquid penetrates into the plurality of openings in the center surface where the liquid may be heated and vaporized.

Liquid tends, for example via capillary forces, to pass between a heater substrate and side portions of a filament arrangement radially outwardly of the heater. This effect may be substantial when using foils as contact portions as in prior art filament arrangements.

By providing a plurality of openings in the side surfaces, the liquid will enter into the openings and thus be kept in the side surfaces.

In the heater assembly according to the invention, leakage may be prevented or reduced.

Having a specific ratio of open area in center surface to side surfaces and in particular a limited open area in the two side surfaces has the further benefit of limiting dissipation of heat from the center surface to the side surfaces. By this, heat may be kept in the center of the heater assembly where evaporation takes place. Overall power consumption of a heater or a respective aerosol-generating device may be limited. In addition, any possibly present overmoulding material in side surfaces of a heater assembly is less affected by heat.

The heater assembly may further comprise a first transition surface being an area of the heater assembly extending between two lines lying perpendicular to the longitudinal axis and crossing the longitudinal axis at two points arranged on the longitudinal axis, one of the two points being situated at a distance from the first contact point equal to about 20 percent and the other one of the two points being situated at a distance from the first contact point equal to about 40 percent of the distance between the first and the second contact point. The heater may yet further comprise a second transition surface being an area of the heater assembly extending between two lines lying perpendicular to the longitudinal axis and crossing the longitudinal axis at two points arranged on the longitudinal axis, one of the two points being situated at a distance from the first contact point equal to about 60 percent and the other one of the two points being situated at a distance from the first contact point equal to about 80 percent of the distance between the first and the second contact point. The first transition surface comprises a plurality of openings defining an open area T1OA, and the second transition surface comprises a plurality of openings defining an open area T2OA.

A ratio of the percentage of the total area of the first transition surface comprising the open area of the first transition surface to the percentage of the total area of the first side surface comprising the open area of the first side surface T1OA/S1OA may be between 1 and 30, and a ratio of the percentage of the total area of the second transition surface comprising the open area of the second transition surface to the percentage of the total area of the second side surface comprising the open area of the second side surface T2OA/S2OA may be between 1 and 30.

A ratio of the open area of the first transition surface to the open area of the first side surface T1OA/S1OA may be between 1 and 30, and a ratio of the open area of the second transition surface to the open area of the second side surface T2OA/S2OA may be between 1 and 30. Preferably, the ratio of the open area of the first transition surface to the first surface or of the second transition surface to the second surface, T1OA/S1OA or T2OA/S2OA is between 2 and 28, for example between 2 and 15 or between 15 and 28.

A transition surface may be arranged between each of the two side surfaces and the center surface. Each transition surface may comprise an open area, for example a gradient, ranging from an open area of a side surface to an open area of the center surface. By the provision of a transition surface, for example by the provision of a gradient in open area, for example realized by a gradient in a mesh density of a mesh filament, a smooth transition of power distribution over the mesh or of the electrical resistance and respective resistive heating in the heater assembly may be achieved.

A transition surface may comprise a small open area, which amount of open area of the transition surface is closer to the open area of a side surface than of the center surface. Thus, in a mesh arrangement, a transition surface may comprise high mesh densities close to the mesh densities of the side surface. In such transition surfaces little heating occurs and heating is concentrated to the center surface.

To enlarge a heating region, the transition surfaces may comprise a large open area, similar or identical to the open area of the center surface.

The first and second transition surface may each extend over about 20 percent of the size of the filament arrangement between two contact points of the heater assembly.

In the heater assembly according to the invention, a center resistance Rc is the electrical resistance of the center surface along the longitudinal axis, a first resistance R1 is an electrical resistance of the first side surface along the longitudinal axis, and a second resistance R2 is an electrical resistance of the second side surface along the longitudinal axis. A ratio of the center resistance to the first resistance Rc/R1 may be between 2 and 400, and a ratio of the center resistance to the second resistance Rc/R2 may be between 2 and 400.

Preferably, the ratio of the center resistance to the first resistance Rc/R1 is between 2 and 300, more preferably between 40 and 200.

Preferably, the ratio of the center resistance to the second resistance Rc/R2 is between 2 and 300, more preferably between 40 and 200. The heater assembly comprises a total resistance Rt corresponding to the electrical resistance between the first contact point and the second contact point.

Preferably, a ratio of the center resistance to the total resistance Rc/Rt corresponds to at least 0.3 or 0.4 preferably 0.5 or 0.6 or 0.7.

Preferably, a ratio of the first resistance to the total resistance R1/Rt is between 0.005 and 0.125, preferably above 0.01, more preferably between 0.01 and 0.1, even more preferably between 0.05 and 0.1.

Preferably, a ratio of the second resistance to the total resistance R2/Rt is between 0.005 and 0.125, preferably above 0.01, more preferably between 0.01 and 0.1, even more preferably between 0.05 and 0.1.

Preferably, the center resistance Rc corresponds to at least 50 percent of a total electrical resistance of the heater assembly between the first and second contact points. Preferably, the first and second resistance each correspond to a maximum of about 13 percent of the total electrical resistance and to a minimum of about 0.5 percent of the total electrical resistance Rt.

The center resistance may correspond up to about 99 percent of the total resistance Rt. Preferably, the center resistance corresponds to about 80 percent to about 98 percent, more preferably to about 90 percent to about 95 percent of the total resistance Rt. Such high electrical resistance in one selected portion of the filament arrangement allows targeted heating of the filaments in this heating region and efficient evaporation of an aerosol-forming fluid to be evaporated.

The regions between the first and second contact point comprising the relatively low first and second resistance R1, R2 define electrical contact regions of the heater assembly. The contact regions are designed to not, or not substantially, transform current flowing through the contact regions of the filament arrangement into heat. The center region between the first and second contact point comprising the relatively high center resistance defines a heating region of the heater assembly.

The ratio of electrical resistance between center resistance and first and second resistance in the ranges defined above, in particular, a low electrical resistance close to the first and second contact points corresponding to a maximum of about 13 percent each of the total electrical resistance and at the same time to a minimum of about 0.5 percent of the total electrical resistance has been found to be beneficial to the performance of a heater assembly.

The low electrical resistance close to the contact points is preferably much smaller than the electrical resistance of the heating region. The electrical resistance close to the contact points may also have a defined minimum.

A low electrical resistance close to the contact points may positively influence an electrical contact of the heater assembly compared to, for example, heater assemblies comprising filament arrangements comprising meshes having low mesh densities, for example, like meshes preferred for heating regions of a filament arrangement. In addition, the low electrical resistance provides good transport of a heating current to the more central heating region, where heating is desired. On the other hand, having a specific ratio of center resistance to first and second resistance, in particular a minimum electrical resistance in contact regions has the benefit of limiting dissipation of heat from the heating region to the contact regions. By this, heat may be kept in a center portion or center surface of a heater assembly where evaporation takes place. Overall power consumption of a heater or a respective aerosol-generating device may be limited. In addition, any possibly present overmoulding material in contact regions, typically a polymer material, is less affected by heat.

The heater assembly according to the invention may have a total resistance Rt between about 0.5 Ohm and about 4 Ohm, more preferably between about 0.8 Ohm and about 3 Ohm, even more preferably about about 2.5 Ohm.

Preferably, the center resistance Rc of the center surface is higher than about 0.5 Ohm, more preferably higher than about 1 Ohm, even more preferably about 2 Ohm.

Preferably, the first resistance R1 of the first side surface is lower than about 100 mOhm, more preferably lower than about 50 mOhm, for example the first resistance is between about 5 mOhm and about 25 mOhm. Preferably, the first resistance is higher than about 3 mOhm, more preferably higher than about 5 mOhm.

Preferably, the second resistance R2 of the second side surface is lower than about 100 mOhm, more preferably lower than about 50 mOhm, for example the resistance is between about 5 mOhm and about 25 mOhm. Preferably, the second resistance is higher than about 3 mOhm, more preferably higher than about 5 mOhm.

In some embodiments of the electrically conductive flat filament arrangements, the center portion of the filament arrangement may be identical to a center surface of a heater assembly. The side portions of the filament arrangement may be identical to the side surfaces of the heater assembly. However, depending on the positions of contact points on the filament arrangement a distance between contact points is smaller than an overall longitudinal extension or length of a filament arrangement.

The distance between the contact points may be equal to a total length of the filament arrangement. Typically, the distance between two contact points is shorter than the total length of the filament arrangement. Preferably, the specification of the remaining portions of the filament arrangement longitudinally extending beyond the contact points is equal or similar to the specifications of the side surfaces and as described herein, in particular relating to resistance and open area.

An open area of side portions of the filament arrangement is each smaller than the open area of the center portion. However, the open areas of the side portions also have a minimum value of about 3 percent and a value of smaller than about 40 percent of the total area of the side portion.

Advantages of the filament arrangement relating to open areas in the form of a plurality of openings, ratios of open areas in a heating region versus open areas in contact regions have already been described relating to the heater assembly according to the invention.

By providing a plurality of openings in the side portions, also an overmoulding of contact portions is facilitated. Overmoulding is typically used for stability purposes of contact portions, for example when using thin contact foils or loose meshes. Side portions or at least portions of side portions may be overmoulded, for example with a heat resistive polymer. Overmoulding may prevent displacement of individual filaments, or an unravelling of filament edges. With an overmoulding of side portions stability of the side portions may be enhanced. This may facilitate mounting of the filament arrangements when assembling a heater assembly. It may also facilitate keeping a form and shape of the filament arrangement. Reproducibility and reliability of heaters using a filament arrangement may thus be improved.

An overmoulding material may be any material suitable for use in a fluid permeable heater according to the invention. An overmoulding material may for example be a material that is able to tolerate high temperatures (in excess of about 300 degree Celsius), for example polyimide or thermoplastics such as for example polyetheretherketone (PEEK).

In the filament arrangement of the present invention, the overmoulding material may penetrate into the openings in the side portions. The openings may, for example, form microchannels in the filament arrangement. Thus, a connection between the material of the filament arrangement and the overmoulding material may be enhanced. The low value of open area, in particular small sized openings, may additionally support that the overmoulding material is kept in the side portions and does not flow through.

With the filament arrangement according to the invention, leakage may be prevented or reduced also with overmoulded side portions. Due to a surface of the overmoulded side portion not being flat, surface irregularities may serve as liquid retention.

The term 'flat' filament arrangement or heater assembly is used throughout the specification to refer to a filament arrangement or a flat heater assembly that is in the form of a substantially two dimensional topological manifold. Thus, the flat filament arrangement and flat heater assembly extend in two dimensions along a surface substantially more than in a third dimension. In particular, the dimensions of the flat filament arrangement in the two dimensions within the surface is at least 5 times larger than in the third dimension, normal to the surface. An example of a flat filament arrangement and a flat heater assembly is a structure between two substantially parallel imaginary surfaces, wherein the distance between these two imaginary surfaces is substantially smaller than the extension within the surfaces. In some preferred embodiments, the flat filament arrangement and the flat heater assembly is planar. In other embodiments, the flat filament arrangement and the flat heater assembly are curved along one or more dimensions, for example forming a dome shape or bridge shape.

A flat filament arrangement is preferably used in a flat heating element, which can be easily handled during manufacture and provides for a robust construction.

The term 'filament' is used throughout the specification to refer to an electrical path arranged between two electrical contacts. A filament may arbitrarily branch off and diverge into several paths or filaments, respectively, or may converge from several electrical paths into one path. A filament may have a round, square, flat or any other form of cross-section. A filament may be arranged in a straight or curved manner.

The term 'filament arrangement' is used throughout the specification to refer to an arrangement of one or preferably a plurality of filaments. The filament arrangement may be an array of filaments, for example arranged parallel to each other. Preferably, the filaments may form a mesh. The mesh may be woven or non-woven. Preferably, the filament arrangement has a thickness of between about 0.5 micrometers and about 500 micrometers. The filament arrangement may, for example, be in the form of an array of parallel or crosswise electrically conductive filaments. The filament may be integrally formed with electrical contacts, for example formed from an electrically conductive foil, for example, stainless steel foil, that is etched to define the filaments or openings in the center portion as well as in the side portions or in the center surface and side surfaces, respectively.

The center portion of a filament arrangement is always arranged in between the two side portions of the filament arrangement. Preferably, the center portion is arranged in the middle between the two side portions. In a filament arrangement having a longitudinal extension larger than a transverse extension such as, for example a rectangular shaped filament arrangement, the center portion also has a longitudinal or rectangular shape. The two side portions may then be arranged adjacent two opposite sides of the center portion and separated from each other by the center portion. For example, in a more circular-shaped filament arrangement, the two side portions may be separated from each other by a centrally arranged center portion and gaps extending from the centrally arranged center portion to a circumference of the circular-shaped filament arrangement.

A ratio of open areas or a value of an open area in the center portion of a filament arrangement may be defined and chosen according to a desired evaporation result or, for example, according to parameters of a heater assembly or of an aerosol-generating device the heater assembly is to be used with. For example, the value of the open area in the center portion, or the number, sizes and arrangement of the openings of the plurality of openings in the center portion may be chosen according to a liquid to be evaporated (viscosity, evaporation temperature, amount of evaporated substance etc.).

A ratio of open areas or a value of an open area in the two side portions of a filament arrangement may be selected according to a heating regime through the filament arrangement or according to the way of contacting the filament arrangement to a heater substrate or contacting the heater assembly. The value of an open area in the two side portions may also be selected, for example, according to an overmoulding material used (flow speed, temperature during overmoulding etc.).

The plurality of openings in the side portions may be arranged homogenously and regularly over each of the two side portions.

The plurality of openings in the side portions may be arranged irregularly over each of the side portions. For example, more or larger openings may be provided in edge regions and smaller or fewer openings may be provided in a central region of the side portion.

Amount and distribution of openings in the two side portions may be identical or symmetric with respect to the center portion. However, amount and distribution of openings in the two side portions may be different in the two side portions. Depending on an arrangement of the filament arrangement in view of a voltage applied (the side portions being connected to ground or to voltage), there may be slightly different local heating. Different amount of openings or, for example, different wire densities in the side portions may be used to even out differences in heating and thus equilibrate temperature variation over a filament arrangement. Consistent heating over an entire heating region of the filament arrangement may thus be supported.

A ratio of open areas or a value of an open area in the center surface of a heater assembly may be defined and chosen according to a desired evaporation result or, for example, according to parameters of a heater assembly or of an aerosol-generating device the heater assembly is to be used with. For example, the value of the open area in the center surface, or the number, sizes and arrangement of the openings of the plurality of openings in the center surface may be chosen according to a liquid to be evaporated (viscosity, evaporation temperature, amount of evaporated substance etc.).

A ratio of open areas or a value of an open area in the two side surfaces of a heater assembly may be selected according to a heating regime through the filament arrangement or according to the way of contacting the filament arrangement to a heater substrate or contacting the heater assembly. The value of an open area in the two side surfaces may also be selected, for example, according to an overmoulding material used (flow speed, temperature during overmoulding etc.).

The plurality of openings in the side surfaces may be arranged homogenously and regularly over each of the two side surfaces.

The plurality of openings in the side surfaces may be arranged irregularly over each of the side surfaces. For example, more or larger openings may be provided in edge regions and smaller or fewer openings may be provided in a central region of the side surfaces.

Amount and distribution of openings in the two side surfaces may be identical or symmetric with respect to the center surface. However, amount and distribution of openings in the two side surfaces may be different in the two side surfaces. Different amount of openings or, for example, different wire densities in the side surfaces may be used to even out differences in heating in the center surface and thus equilibrate temperature variation over a total heater surface. Consistent heating over at least a central surface of the filament arrangement may thus be supported.

In the flat filament arrangement, a transition portion may be arranged between each of the two side portions and the center portion. Each transition portion comprises a plurality of openings defining an open area, for example a gradient, ranging from an open area of a side portion to an open area of the center portion. The distribution of the open area across the transition portion may vary between the side portion and the center portion. By the provision of a transition portion, for example by the provision of a gradient in open area, for example realized by a gradient in a mesh density of a mesh filament, a smooth transition of power distribution over the mesh or of the electrical resistance and respective heating may be achieved.

The transition portions may correspond in size, open areas and physical characteristics to the transition surfaces of a heater assembly.

If a direction extending from side portion to side portion of a filament arrangement is defined as longitudinal direction of the filament arrangement, and a transition portion is each arranged between the two side portions and the center portion, an extension of a transition portion is about 20 percent of a total longitudinal extension of the filament arrangement along the longitudinal direction.

The flat filament arrangement and the heater assembly may also comprise variations in the open area, such as for example number or size of openings, in one or all of the center portion or center surface, the side portions or side surfaces, and the transition portions or transition surfaces relative to the longitudinal axis or the longitudinal direction of the heater assembly or filament arrangement, respectively.

The filament arrangement may, for example, comprise a central longitudinal region extending from one of the two side portions over the central portion to the other one of the two side portions. The heater assembly may, for example, comprise a central longitudinal region extending from one of the two side surfaces over the transition surface, the central surface, the second transition surface to the other one of the two side portions. Therein, the percentage of the total area of the center portion inside the central longitudinal region comprising open area may be less than the percentage of the total area of the center portion outside of the central longitudinal region comprising open area. Therein, an open area in the central longitudinal region may be smaller than an open area outside of the central longitudinal region. For example, more or larger openings may be arranged in edge regions along the filament arrangement than in the central longitudinal region. For example, a mesh density may be higher in the central longitudinal region than in lateral longitudinal regions along the filament arrangement. By this, a power distribution may be concentrated onto a central region of the central portion or central surface. Such a specific power distribution may, for example, be realized by a flat filament arrangement wherein in a longitudinal direction of the filament arrangement more filaments are arranged in the central longitudinal region than outside the central longitudinal region.

The flat filament arrangement may, for example, be a perforated sheet. The center portion of the perforated sheet may comprise a plurality of heater filaments separated or distanced from each other by a plurality of openings. The side portions of the perforated sheet each comprise a plurality of openings.

The openings may, for example, be manufactured by chemical etching or laser treatment.

The flat filament arrangement may, for example, be a mesh arrangement, wherein a mesh of the center portion and meshes of the first and second side portion each comprise a mesh density. The mesh density in the center portion is lower than the mesh density in each of the first and second side portions. Interstices between filaments of the meshes define the open area of the center portion and the open areas of each of the two side portions.

Mesh arrangements may be manufactured by weaving applying different weaving modes to manufacture the different portions of the mesh. By this, a single strip or a continuous band of mesh may be manufactured having different density meshes in the side portion and the center portion or in a center surface and two side surfaces. A continuously produced band of mesh may be cut to appropriately sized strips of mesh.

The filament arrangement may be manufactured at low cost, in a reliable and repeatable manner. The filament arrangement may be manufactured in one manufacturing step, not requiring assembly of individual filament arrangement parts.

In a mesh arrangement, a mesh density gradient may be located between the first portion and the center portion and between the center portion and the second side portion. These mesh gradients represent transition portions between center portion and side portions.

The mesh may be a woven mesh. The mesh of the center portion and center surface may comprise a weft aperture having a same size than a warp aperture of the mesh of the center portion or center surface. By this a mesh having regular square-shaped openings in the center portion and center surface may be manufactured.

The meshes of the two side portions and side surfaces may comprise a weft aperture larger than zero and no warp aperture. By this, very small, regularly arranged openings in the meshes of the side portions and side surfaces may be manufactured.

Preferably, in weaving direction of the filament arrangement a same number of (warp) filaments are arranged next to each other along an entire length of the filament arrangement. In these embodiments, continuing warp filaments extend preferably at least from a first side surface to the second side surface, and more preferably along the entire arrangement from one side portion over the center portion to the second side portion. By this method, mesh arrangements may be manufactured, wherein a warp aperture in the two side portions is equal to the warp aperture of the center portion or a warp aperture in the two side surfaces is equal to the warp aperture of the center surface.

Preferably, the filament arrangement is a mesh arrangement.

For the filaments of the filament arrangement any electrically conductive material suitable for manufacturing a filament arrangement and for being heated may be used.

Preferred materials for the filament arrangement are metals, including metal alloys, and carbon fibers. Carbon fibers may be added to metals or other carrier material to vary the resistance of the filaments.

Filament diameters may be in a range between about 8 micrometer and about 50 micrometer, preferably between about 10 micrometer and about 30 micrometer, more preferably between about 12 micrometer and about 20 micrometer, for example about 16 micrometer.

Side portions made of mesh may be compressed. By this, electrical contact between individual filaments of the mesh and thus of the side portions of the filament arrangement may be improved.

Sizes of openings in the center portion or center surface may, for example, have a length and width or diameter between about 25 micrometer and about 75 micrometer, for example a length and width or diameter between about 60 and about 80 micrometer.

Sizes of openings in the side portions or side surfaces may, for example have length and width between about 0.5 micrometer and about 75 micrometer. Preferably, sizes of openings in side portions and side surfaces have, for example, a width up to about 75 micrometer when a length decreases versus about 0.5 micrometer. Preferably, sizes of openings in side portions and side surfaces have diameters between about 5 micrometer and about 50 micrometer or corresponding opening areas.

The center portion of the flat filament arrangement may have a size in a range, for example, between about 5 mm² and about 35 mm², for example in a range between about 10 mm² and about 30 mm², for example about 25 mm². Preferably, a center portion has a rectangular, preferably substantially square form, for example about 5x5 mm². Heat dissipation may be kept low in portions having about a same length and width.

The center surface of the heater assembly may have a size in a range, for example, between about 5 mm² and about 35 mm², for example in a range between about 10 mm² and about 30 mm², for example about 25 mm². Preferably, a center surface has a rectangular, preferably substantially square form, for example about 5x5 mm². Heat dissipation may be kept low in surfaces having about a same length and width.

Throughout this application, whenever a value is mentioned, this is to be understood such that the value is explicitly disclosed. However, a value is also to be understood as not having to be exactly the particular value due to technical considerations.

A side portion of a filament arrangement may have a size, for example in a range between about 3 mm² to about 15 mm², for example in a range between about 5 mm² to about 10 mm², for example about 5 mm² or about 10 mm².

A side surface of a heater assembly may have a size, for example in a range between about 3 mm² to about 15 mm², for example in a range between about 5 mm² to about 10 mm², for example about 5 mm² or about 10 mm².

Preferably, side portions and side surfaces have the form of strips, for example a rectangular strip of about 5x(1-2) mm².

The sizes of contact portions or side portions and side surfaces, respectively, may be adapted to provide good contact with connectors used to connect the heater assembly to a power supply, for example a contact with pogo pins.

A number of openings of the plurality of openings in the center portion may, for example, be in a range between about 5 and about 100 openings per mm², preferably between about 15 and about 70 openings per mm², for example about 40 openings per mm².

A number of openings of the plurality of openings in the center surface may, for example, be in a range between about 5 and about 100 openings per mm², preferably between about 15 and about 70 openings per mm², for example about 40 openings per mm².

A number of openings of the plurality of openings in a side portion may, for example, be in a range between about 20 and about 400 openings per mm², preferably between about 50 and about 350 openings per mm², for example about 300 to about 350 openings per mm².

A number of openings of the plurality of openings in a side surface may, for example, be in a range between about 20 and about 400 openings per mm², preferably between about 50 and about 350 openings per mm², for example about 300 to about 350 openings per mm².

A filament arrangement may be pretreated. Pretreatment may be a chemical or physical pretreatment, for example, changing the surface characteristic of the filament surface. For example, a filament surface may be treated to enhance wettability of the filament, preferably in a center portion or a center surface only. Increased wettability has been found particularly favorable for liquids typically used in electronic vaporization devices, so called e-liquids. E-liquids typically comprise an aerosol-former such as glycerol or propylene glycol. The liquids may additionally comprise flavourants or nicotine.

The aerosol-forming liquids evaporated by a heated filament arrangement according to the invention comprises at least one aerosol former and a liquid additive.

The aerosol-forming liquid may comprise water.

The liquid additive may be any one or a combination of a liquid flavour or liquid stimulating substance. Liquid flavour may for example comprise tobacco flavour, tobacco extract, fruit flavour or coffee flavour. The liquid additive may, for example, be a sweet liquid such as for example vanilla, caramel and cocoa, a herbal liquid, a spicy liquid, or a stimulating liquid containing, for example, caffeine, taurine, nicotine or other stimulating agents known for use in the food industry.

The flat filament arrangement may have a total electrical resistance between about 0.5 Ohm and about 4 Ohm, more preferably between about 0.8 Ohm and about 3 Ohm, even more preferably about 2.5 Ohm.

Preferably, the electrical resistance of the center portion is higher than about 0.5 Ohm, more preferably higher than 1 Ohm, even more preferably about 2 Ohm.

Preferably, the electrical resistance of each of the first and second side portions is lower than about 100 mOhm, more preferably lower than about 50 mOhm, for example the resistance is between about 5 mOhm and about 25 mOhm. Preferably, the electrical resistance of each of the first and second side portions is higher than about 3 mOhm, more preferably higher than about 5 mOhm.

Due to the open structure over an entire filament arrangement, resistance of the filament arrangement is different to, for example, prior art mesh filaments, where a homogenous mesh with a same mesh density over the entire filament arrangement is mounted to a heater assembly or where a filament arrangement is comprised of a mesh with two side metal plates as contacts. Due to the defined open area in the side portions, a resistance over the filament arrangement may be optimized in view of contacting and heating of the filament arrangement as well as in view of assembly and use of a heater assembly comprising the filament arrangement.

The filament arrangement according to the invention may be characterized by its resistance. The resistance in contact regions is higher than when using metal plates as contacts but may be the same or higher in a heating region, depending on, for example, a mesh density used for the central heating portion.

Advantageously, the fluid permeable heater assembly for aerosol-generating systems comprises a substrate comprising an opening through the substrate. The electrically conductive flat filament arrangement according to the invention and as described herein extends over the opening in the substrate. The heater assembly further comprises fastener attaching the flat filament arrangement to the substrate.

The fastener may itself be electrically conductive and may serve as electrical contact for providing heating current through the filament arrangement.

The fastener may be chemical or mechanical fastener. The filament arrangement may, for example be attached to the substrate by bonding or gluing.

Preferably, the fastener is a mechanical fastener such as clamps, screws or form-locking fastener. Clamps and flat heater assemblies using clamps to clamp a filament arrangement to a heater substrate have been described in detail in the international patent publication WO2015/117701. Reference is herewith made to this international patent publication WO2015/117701 and its content relating to the heater assembly and clamps used and described therein is incorporated herewith.

The fastener may be one or a combination of the aforementioned fastener.

Preferably, the heater assembly is a flat heater assembly, preferably a resistively heatable fluid permeable flat heater assembly.

According to the invention, there is also provided an electrically operated aerosol-generating system. The system comprises an aerosol-generating device and a cartridge comprising a liquid aerosol-forming substrate. The system further comprises a fluid permeable heater assembly according to the invention and as described herein or a fluid permeable heater assembly comprising a flat filament arrangement according to the invention and as described herein for heating liquid aerosol-forming substrate.

The cartridge comprises a housing having an opening, with the heater assembly extending across the opening of the housing of the cartridge. The aerosol-generating device comprises a main body defining a cavity for receiving the cartridge, an electrical power source, and electrical contacts for connecting the electrical power source to the heater assembly for heating the filament arrangement.

Preferably, the cartridge comprises a liquid comprising at least an aerosol-former and a liquid additive.

Features and advantages of the aerosol-generating system have been described relating to the filament arrangement according to the invention and relating to the heater assembly according to the invention.

The invention is further described with regard to embodiments, which are illustrated by means of the following drawings, wherein:
- Fig. 1: is a schematic illustration of a mesh arrangement;
- Fig. 1a: is another schematic illustration of the mesh arrangement of Fig. 1;
- Fig. 2: is an exploded view of a heater assembly with mesh arrangement;
- Fig. 3: shows the assembled mesh heater assembly of Fig. 2;
- Fig. 4: shows a heater substrate with mesh arrangement;
- Fig. 5: is an enlarged view of Fig.4;
- Fig. 6: shows enlarged views of transition and contact portions of a mesh arrangement;
- Fig. 7: shows a tin-plated contact portion of a mesh heater;
- Fig. 8: is a schematic illustration of another embodiment of a mesh arrangement;
- Fig. 9: is a schematic illustration of a mesh density of a heater assembly between two contact points on a filament arrangement, for example the filament arrangement of Fig. 1;
- Fig. 9a: is a schematic illustration of a mesh density of a filament arrangement, for example of Fig. 1;
- Fig. 10: is a schematic illustration of resistance distribution over a heater assembly.

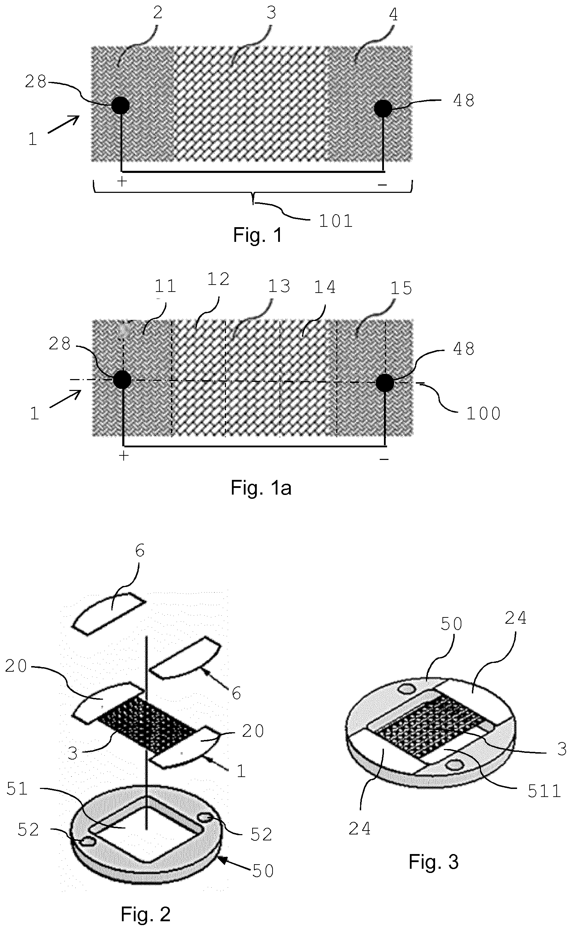

In **Fig. 1** a mesh arrangement 1 for a resistively heatable flat fluid permeable heater is shown. The mesh arrangement has a rectangular shape having a length 101 (Lf) and comprises a first side portion 2, a center portion 3 and a second side portion 4. The first and second side portions 2, 4 are arranged on opposite sides of the center portion 3. The center portion is designed to be the main heating region of the mesh arrangement. In Fig. 1 the three portions of the mesh filament have a rectangular shape and the two side portions 2, 4 have a same size.

The meshes defining the first and the second side portions 2, 4 have a higher density than the mesh defining the central portion 3. Preferably, the densities of the meshes of the side portions are identical. The meshes of the side portions have an open area formed by the sum of the interstices between the filaments of the meshes of preferably less than 20 percent of the total area of each of the first and second side portion. Thus, in the first and second side portion 2,4 an open area is each preferably about maximal 1 mm², with a total size of each of the first and second side portion of about 5 mm².

The side or contact portions may each be contacted, for example by a pogo pin, in one spot as indicated by contact points 28, 48. Over the contact points 28, 48 a voltage is applied. The current flowing between the side portions 2, 4 causes resistive heating of the mesh filament in the center portion 3 according to its high center resistance.

In **Fig. 1a** the same mesh arrangement 1 as in Fig. 1 is shown. When arranged in a heater assembly and contacted in contact points 28, 48, areas of the filament arrangement define heater surfaces each extending over 20 percent of the distance between the first contact point 28 and the second contact point 48.

A longitudinal axis 100 is defined between the first and second contact point 28, 48, which longitudinal axis corresponds to a central longitudinal axis of the filament arrangement 1. Along the longitudinal axis 100 the resistance of the heater surface is measured (see further below).

A first surface 11 extends from the first contact point 28 over 20 percent of the distance between first and second contact point 28, 48 along the longitudinal axis into the direction of the second contact point.

A first transition surface 12 extends between 20 percent and 40 percent of the distance between first and second contact point 28, 48 along the longitudinal axis.

A center surface 13 extends between 40 percent and 60 percent of the distance between first and second contact point 28, 48 along the longitudinal axis.

A second transition surface 14 extends between 60 percent and 80 percent of the distance between first and second contact point 28, 48 along the longitudinal axis.

A second side surface 15 extends between 80 percent and 100 percent of the distance between first and second contact point 28, 48 along the longitudinal axis counted from the first contact point into the direction of the second contact point.

The center surface 13 comprises a low mesh density over its entire surface.

The first and second side surfaces 11, 15 comprise a high mesh density over its entire surface.

The first and second transition surfaces 12, 14 comprise parts with a high mesh density and parts with a low mesh density.

**Fig. 2** and **Fig. 3** schematically show an example of a setup of a flat, fluid-permeable heater assembly with a mesh arrangement. In the exploded view of the heater in Fig. 2 an electrically insulating substrate 50, a heater element and filament arrangement in the form of a mesh arrangement 1 and two metal sheets 6 are shown. The metal sheets may, for example, be sheets of tin, to alter electrical contact of connectors, for example contact pins, with the side portions 20 of the mesh arrangement 1.

The substrate 50 has the form of a circular disc and comprises a centrally arranged opening 51. The substrate comprises two bore holes 52 arranged diagonally opposite each other in the substrate. The bore holes 52 may serve for positioning and mounting the heater assembly for example in an aerosol-generating device.

The mesh arrangement 1 comprises a central portion 3 and in the embodiment shown in Figs. 2 and 3 two PEEK overmoulded side portions 20. The mesh arrangement is arranged over the square-formed centrally arranged opening 51 and over the substrate 50. The entire central portion 3 of the mesh arrangement comes to lie over the opening 51. The two side portions 20, in particular those portions of the side portions overmoulded with PEEK and tin-plated (covered with the metal sheets 6) come to lie on the substrate 50.

The width of the mesh of the central portion 3 is smaller than the width of the opening 51 such that on both lateral sides of the central portion 3 an open portion 511 of the opening 51 is formed. The open portions are not covered by mesh. The tin-plated dense mesh of the side portions forms a more plane contact area 24 than the mesh itself. The contact area 24 is arranged parallel to the top surface of the substrate 50 of the heater assembly. The contact areas 24 are for contacting the heater assembly by an electrical connector from for example a battery.

Fig. 3 shows the heater assembly of Fig. 2 in an assembled state. The mesh arrangement 1 may be attached to the substrate 50 by mechanical means or for example by adhesive.

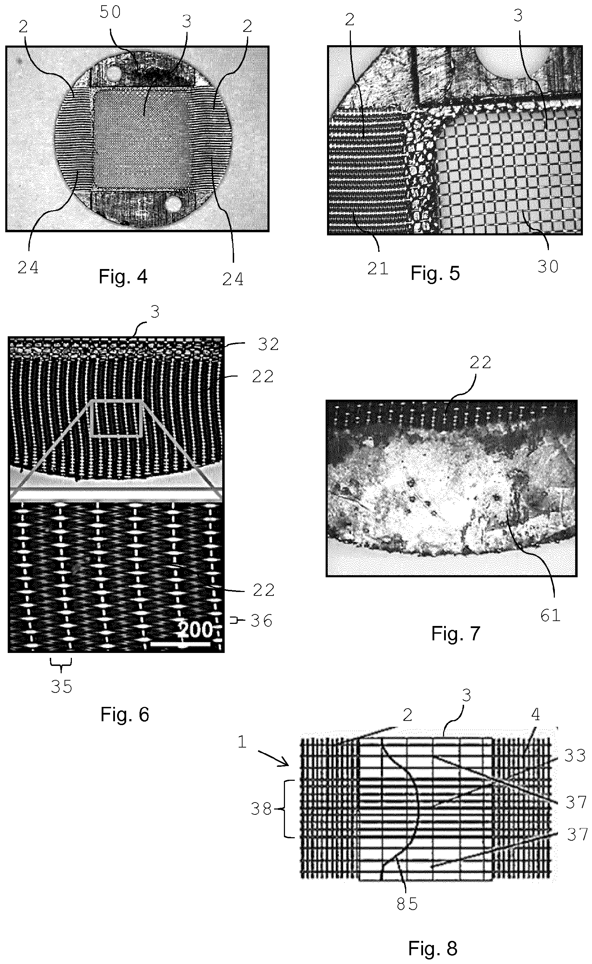

**Fig. 4** shows a heater substrate 50 with a mesh arrangement 1 attached thereto. The mesh arrangement is a rectangular strip of mesh with a high density mesh in contact areas 24 of the heater assembly and a low density mesh in between defining the heating region of the heater assembly.

This may better be seen in **Fig. 5****,** which is an enlarged view of a detail of Fig.4. The low density mesh of the center portion of the mesh arrangement has rectangular interstices 30 in a micrometer range, for example 70 micrometer. With a wire diameter of the filaments of 16 micrometer, the open area of the center portion covers about 75 percent of the total area of the center portion.

The high density mesh of the side portions 2 of the mesh arrangement has smaller interstices 21 of about 0.1 micrometer x 5 micrometer. With a filament diameter of 16 micrometer, the open area of the side portions covers about 3 percent of the total area of each of the side portions.

The mesh arrangement has been produced in one piece by different weaving modes.

The amount of filaments in a weaving direction is identical over the entire filament arrangement. The weaving direction corresponds to the warp direction of the filament arrangement, which warp direction corresponds to the main current flow direction in the mesh arrangement. However, the weaving density of the filaments in weft direction (perpendicular to the warp direction) is enhanced in the side portions 2. A distance between filaments in the weft direction may be reduced to zero in the side portions 2, 4.

No transition portion between side portion 2 and center portion 3 is present in the mesh of Fig. 4 and 5. Depending on the production mode, a transition portion may comprise a density gradient in mesh density. Preferably, such density gradient smoothly changes from the low density of the mesh of the center portion to the higher density of the mesh of the side portion and vice versa.

In **Fig. 6** a small transition portion 32 between the center portion 3 and a compressed side portion 22 is shown.

The higher density mesh in the side portions 22 has been compressed to improve electrical contact between the individual filaments of the mesh. A filament to filament distance between warp filaments 35 is about 25 micrometer to 75 micrometer, in Fig. 6 about 70 micrometer. The filament to filament distance of weft filaments 36 is zero. The open area in the side portions is generated by the manufacturing of the filament arrangement through weaving.

To improve electrical contact of the side portion 22, an outermost part of the compressed side portion 22 is tin-plated 61 as may be seen in **Fig. 7****.**

**Fig. 8** shows a mesh arrangement 1 having a first side portion 2, an intermediate central portion 3 and an opposite second side portion 4. The mesh density in the two side portions 2, 4 is higher than the mesh density in the center portion 3. The mesh arrangement 1 comprises a longitudinal central portion 38 arranged along a longitudinal central axis of the mesh arrangement 1. The mesh density in this longitudinal central portion 38 is higher than outside in lateral side regions 37 of the mesh arrangement. The longitudinal central portion 38 has a width of about 50% to 60% of the total width of the mesh arrangement 1.

The higher mesh density in a central region 33 of the central portion leads to a high power density in this region and concentrates the main heating zone to this central region 33 of the center portion 3. Due to the different mesh densities in the different regions of the mesh arrangement, the highest power density is in the middle or central region 33 of the center portion 3. The lower density areas in the lateral regions 37 in the central portion 3 have comparably high resistance. The power density curve over the width of the central portion 3 is shown with line 85.

The side portions 2, 4 form high density mesh contact pads with comparably low resistance. The electrical contacts are preferably in a center of the side portions 2, 4, where an electrical resistance is lowest in the side portions.

The examples shown in the figures typically have symmetric side portions with a same size and a same mesh density or density distribution. Such embodiments simplify a manufacturing and symmetric arrangement of a heater assembly. However, asymmetric mesh arrangement portions and mesh gradients may easily be provided to achieve a desired power distribution regime in the mesh filament.

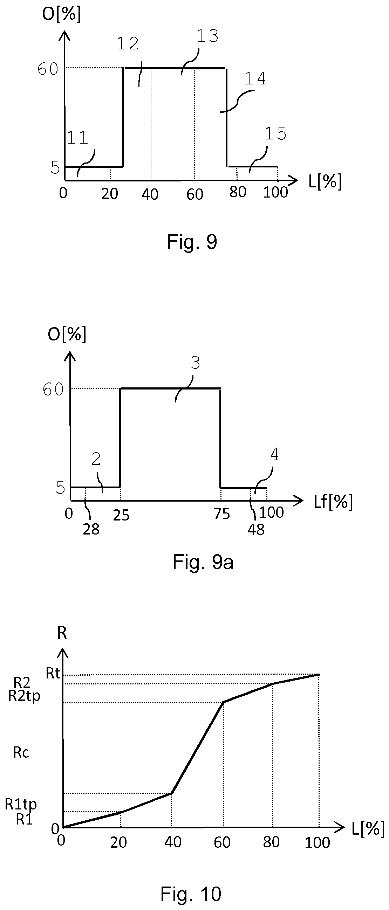

In **Fig. 9** an open area distribution for a heater assembly, for example comprising, the mesh arrangement of Fig.1a, is shown. The vertical axis (O[%]) indicates the open area in the different surfaces of the filament arrangement. The horizontal axis (L[%]) indicates the position on the longitudinal axis 100 from the first contact point to the second contact point.

In a first surface 11 the open area S1OA is low, in Fig. 9 indicated as 5 percent. In the center surface 13 the open area ScOA is high, in Fig. 9 indicated as 60 percent. In the second surface 15 the open area S2OA is low again, in Fig. 9 indicated as 5 percent as in the first surface 11.

In the first transition surface 12, the open area varies over the length of the transition surface 12. At first the open area of the transition surface T1OA is identical to the open area of the first surface S1OA. Then the open area of the first transition surface T1OA is as high as the open area of the center surface ScOA.

The second transition surface 14 arranged between center surface 13 and second surface 15 is symmetric to the first transition surface 12 with respect to the center surface 13. In the second transition surface 14, the open area T2OA also varies over the length of the transition surface 14. At first the open area of the second transition surface T2OA is identical to the open area of the center surface ScOA. Then the open area of the second transition surface is as high as the open area of the second side surface S2OA.

The filament arrangement is defined as having two surfaces 11,15, two transition surfaces 12,14 and a center surface 13 each extending over 20 percent along the longitudinal axis 100 of the filament arrangement.

In **Fig. 9**a an open area distribution of a filament arrangement, for example the mesh arrangement of Fig.1, is shown. The vertical axis indicates the open area in the different portions of the filament arrangement. The horizontal axis (Lf[%]) indicates the position on the longitudinal axis along the length Lf of the filament arrangement.

In a first side portion 2 the open area P1OA is low, in Fig. 9 indicated as 5 percent. In the center portion 3 the open area PcOA is high, in Fig. 9 indicated as 60 percent. In the second side portion 4 the open area P2OA is low again, in Fig. 9 indicated as 5 percent as in the first side portion 2.

The filament arrangement is defined as having two side portions 2, 4 and a center portion 3. The side portions each extend over about 25 percent of the size of the filament arrangement and the center portion extends to about 50 percent of the size of the filament arrangement.

In **Fig. 10** a schematic illustration of an example of a resistance distribution along the longitudinal axis 100 of a heater assembly between a first contact point at position 0% and a second contact point at position 100% is shown. The vertical axis indicates the resistance (R) of the heater assembly up to a total resistance Rt of the heater assembly. The horizontal axis (L[%]) indicates the position on the longitudinal axis from the first contact point to the second contact point.

In the example of Fig. 10, the heater assembly comprises a first resistance R1 which is present over 20 percent along the longitudinal axis starting at the first contact point at 0 into the direction of the second contact point. A first transition resistance R1tp is present between 20 percent and 40 percent along the longitudinal axis. A center resistance Rc is present between 40 percent and 60 percent along the longitudinal axis and after the first contact point. A second transition resistance R2tp is present from a point between 60 percent and 80 percent along the longitudinal axis after the first contact point. A second resistance is present from 80 percent to 100 percent, that is, over the last 20 percent of the heater assembly along the longitudinal axis between the first and second contact point.

The heater assembly is contacted in the first and the second contact points and a current is allowed to flow through the filament arrangement of the heater assembly.

The first resistance R1 may be up to a maximum of 13 percent of the total resistance Rt and as low as 0.5 percent of the total resistance Rt.

The first and second transition resistances R1tp, R2tp are each not higher than the center resistance in order to prevent extensive heating in a transition surface of a heater assembly. Typically, the first and second transition resistance R1tp, R2tp have a value in between the first resistance R1 and the center resistance Rc or the center resistance Rc and the second resistance R2, respectively. The center resistance Rc is about 50 percent of the total resistance Rt of the heater assembly. Preferably, the center resistance Rc is more than 50 percent of the total resistance Rt. The second resistance may be up to a maximum of 13 percent of the total resistance Rt and as low as 0.5 percent of the total resistance Rt.

The first and second resistance R1, R2, the first and second transition resistance R1tp, R2tp and the center resistance Rc add up to the total resistance Rt of the heater assembly.

As becomes obvious from the resistance distribution, side portions of filament arrangements may for example be smaller or larger, have more and smaller or less and larger openings, be smaller and have higher mesh density or be larger and have lower mesh density, all in order to achieve a same or a specific resistance regime in the surfaces of the heater assembly. Such variations allow much flexibility in the application of the filament arrangement and the heater assembly. For example, it enables to adapt the filament arrangement and heater assembly to various liquids to be aerosolized, for example more or less viscous fluids.

The filament arrangement may easily be adapted to differently sized heaters or to aerosol-generating devices having more or less power available for heating a heater comprising the filament arrangement.

## Claims

1. Electrically conductive flat filament arrangement for a fluid permeable heater assembly for aerosol-generating systems, the flat filament arrangement comprising:
a center portion (3) and two side portions (2, 4, 20, 22), the two side portions (2, 4, 20, 22) being arranged on opposite sides of the center portion (3), wherein the center portion (3) defines a heating region of the filament arrangement and the side portions (2, 4, 20, 22) define electrical contact regions of the filament arrangement;
the center portion (3) and the two side portions (2, 4, 20, 22) each comprise a plurality of openings, the plurality of openings of the center area defining an open area of the center portion (3) and the plurality of openings of each side portion (2, 4, 20, 22) defining an open area of the side portions (2, 4, 20, 22);
**characterised in that** the percentage of the total area of the center portion (3) comprising the open area of the center portion (3) is greater than the percentage of the total area of one of the side portions (2, 4, 20, 22) comprising the open area of the side portion (2, 4, 20, 22) .

2. Flat filament arrangement according to claim 1, wherein a ratio of the percentage of the total area of the center portion (3) comprising the open area of the center portion (3) to the percentage of the total area of one of the two side portions (2, 4, 20, 22) comprising the open area of the side portion (2, 4, 20, 22) is between 1.1 and 30.

3. Flat filament arrangement according to claims 1 or 2, wherein the open area of the center portion (3) is between 40 percent and 90 percent of the total area of the center portion (3);
and wherein the open area in each of the two side portions (2, 4, 20, 22) is larger than 3 percent and smaller than 40 percent of each of the total areas of the two side portions (2, 4, 20, 22).

4. Flat filament arrangement according to any one of claims 1 to 3, wherein a transition portion (32) is arranged between each of the two side portions (2, 4, 20, 22) and the center portion (3), each transition portion (32) comprising a plurality of openings defining an open area, and wherein the distribution of the open area across the transition portion (32) varies between the side portion (2, 4, 20, 22) and the center portion (3).

5. Flat filament arrangement according to any one of claims 1 to 4, comprising a central longitudinal region extending from one of the two side portions (2, 4, 20, 22) over the center portion (3) to the other one of the two side portions (2, 4, 20, 22), wherein the percentage of the total area of the center portion (3) inside the central longitudinal region comprising open area is less than the percentage of the total area of the center portion (3) outside of the central longitudinal region comprising open area.

6. Flat filament arrangement according to any one of claims 1 to 5, wherein the filament arrangement is a mesh arrangement (1), wherein a mesh of the center portion (3) and meshes of the first and second side portions (2, 4, 20, 22) each comprise a mesh density, wherein the mesh density in the center portion (3) is lower than the mesh density in each of the first and second side portions (2, 4, 20, 22).

7. Flat filament arrangement according to claim 6, wherein a mesh density gradient is located between the first side portion (2, 4, 20, 22) and the center portion (3) and between the center portion (3) and the second side portion (2, 4, 20, 22).

8. Flat filament arrangement according to any one of claims 6 or 7, wherein the mesh is woven and in weaving direction of the filament arrangement a constant number of filaments are arranged next to each other along the entire length of the filament arrangement.

9. Flat filament arrangement according to any one of claims 6 to 8, comprising a central longitudinal region extending from one of the two side portions (2, 4, 20, 22) over the center portion (3) to the other one of the two side portions (2, 4, 20, 22), wherein the mesh is woven and in weaving direction of the filament arrangement more filaments are arranged in the central longitudinal region than outside the central longitudinal region.

10. Fluid permeable heater assembly for aerosol-generating systems, the fluid permeable heater assembly comprising an electrically conductive flat filament arrangement according to any one of claims 1 to 9, and
a first contact point (28, 48) and a second contact point (28, 48) for electrically contacting the flat filament arrangement, wherein a longitudinal axis is defined between the first contact point (28, 48) and the second contact point (28, 48),
wherein a center surface Sc (10) is an area of the heater assembly extending between two lines lying perpendicular to the longitudinal axis and crossing the longitudinal axis at two points arranged on the longitudinal axis, one of the two points being situated at a distance from the first contact point (28, 48) equal to 40 percent and the other one of the two points being situated at a distance from the first contact point (28, 48) equal to 60 percent of the distance between the first and the second contact point (28, 48) ;
wherein a first side surface S1 (11) is an area of the heater assembly extending between two lines lying perpendicular to the longitudinal axis and crossing the longitudinal axis at the first contact point (28, 48) and a point arranged on the longitudinal axis and situated at a distance from the first contact point (28, 48) equal to 20 percent of the distance between the first and the second contact point (28, 48);
wherein a second side surface S2 (15) is an area of the heater assembly between two lines lying perpendicular to the longitudinal axis and crossing the longitudinal axis at the second contact point (28, 48) and a point arranged on the longitudinal axis and situated at a distance from the first contact point (28, 48) equal to 80 percent of the distance between the first and the second contact point (28, 48) ;
wherein the center surface Sc (10) comprises a plurality of openings defining an open area ScOA, the first side surface S1 (11) comprises a plurality of openings defining an open area S1OA, and the second side surface S2 (15) comprises a plurality of openings defining an open area S2OA, and
wherein the percentage of the total area of the center surface (10) comprising the open area of the center surface (10) is greater than the percentage of the total area of the first side surface (11) comprising the open area of the first side surface (11) and
wherein the percentage of the total area of the center surface (10) comprising the open area of the center surface (10) is greater than the percentage of the total area of the second side surface (15) comprising the open area of the second side.

11. Heater assembly according to claim 10, wherein a ratio of the percentage of the total area of the center surface (10) comprising the open area of the center surface (10) to the percentage of the total area of the center surface (10) comprising the open area of the first side surface (11) ScOA/S1OA is between 1.1 and 30, and wherein a ratio of the percentage of the total area of the center surface (10) comprising the open area of the center surface (10) to the percentage of the total area of the second side surface (15) comprising the open area of the second side surface (15) ScOA/S2OA is between 1.1 and 30.

12. Heater assembly according to claims 10 or 11, wherein the open area of the center surface (10) ScOA is between 40 percent and 90 percent of the total area of the center surface (10);
and wherein the open area in each of the two side surfaces (11, 15) S1OA, S2OA is larger than 3 percent and smaller than 40 percent of each of the total areas of the two side surfaces (11, 15).

13. Heater assembly according to any one of claims 10 to 12, further comprising a first transition surface (12) being an area of the heater assembly extending between two lines lying perpendicular to the longitudinal axis and crossing the longitudinal axis at two points arranged on the longitudinal axis, one of the two points being situated at a distance from the first contact point (28, 48) equal to 20 percent and the other one of the two points being situated at a distance from the first contact point (28, 48) equal to 40 percent of the distance between the first and the second contact point (28, 48); and
a second transition surface (14) being an area of the heater assembly extending between two lines lying perpendicular to the longitudinal axis and crossing the longitudinal axis at two points arranged on the longitudinal axis, one of the two points being situated at a distance from the first contact point (28, 48) equal to 60 percent and the other one of the two points being situated at a distance from the first contact point (28, 48) equal to 80 percent of the distance between the first and the second contact point (28, 48),
wherein the first transition surface (12) comprises a plurality of openings defining an open area T10A, and the second transition surface (14) comprises a plurality of openings defining an open area T2OA,
wherein a ratio of the percentage of the total area of the first transition surface (12) comprising the open area of the first transition surface (12) to the percentage of the total area of the first side surface (11) comprising the open area of the first side surface (11) T1OA/S1OA is between 1 and 30,
wherein a ratio of the percentage of the total area of the second transition surface (14) comprising the open area of the second transition surface (14) to the percentage of the total area of the second side surface (15) comprising the open area of the second side surface (15) T2OA/S2OA is between 1 and 30.

14. Heater assembly according to any one of claims 10 to 13, wherein a center resistance Rc is the electrical resistance of the center surface (10) along the longitudinal axis, wherein a first resistance R1 is an electrical resistance of the first side surface (11) along the longitudinal axis, wherein a second resistance R2 is an electrical resistance of the second side surface (15) along the longitudinal axis;
and wherein a ratio of the center resistance to the first resistance Rc/R1 is between 2 and 400, and wherein a ratio of the center resistance to the second resistance Rc/R2 is between 2 and 400.

15. Heater assembly according to any one of claims 10 to 14, wherein a total resistance Rt corresponding to the electrical resistance between the first contact point (28, 48) and the second contact point (28, 48) is between 0.5 Ohm and 4 Ohm, wherein the center resistance Rc is higher than 0.5 Ohm, wherein the first resistance R1 and the second resistance R2 are each lower than 100 mOhm.

16. Heater assembly according to any one of claims 10 to 15, comprising a substrate comprising an opening through the substrate;
wherein the electrically conductive flat filament arrangement extends over the opening in the substrate; and
a fastener attaching the flat filament arrangement to the substrate.

17. Electrically operated aerosol-generating system comprising:
an aerosol-generating device and a cartridge comprising a liquid aerosol-forming substrate;
a fluid permeable heater assembly according to any one of claims 10 to 16 or
a fluid permeable heater assembly comprising a flat filament arrangement according to any one of claims 1 to 9,
wherein the cartridge comprises a housing having an opening, with the heater assembly extending across the opening of the housing of the cartridge,
and wherein the aerosol-generating device comprises a main body defining a cavity for receiving the cartridge, an electrical power source, and electrical contacts for connecting the electrical power source to the heater assembly.

## Patentansprüche

1. Elektrisch leitfähige, flache Fadenanordnung für eine fluiddurchlässige Heizvorrichtungsbaugruppe für Aerosolerzeugungssysteme, wobei die flache Fadenanordnung aufweist:
einen Mittelabschnitt (3) und zwei Seitenabschnitte (2, 4, 20, 22), wobei die beiden Seitenabschnitte (2, 4, 20, 22) auf gegenüberliegenden Seiten des Mittelabschnitts (3) angeordnet sind, wobei der Mittelabschnitt (3) einen Heizbereich der Fadenanordnung definiert und die Seitenabschnitte (2, 4, 20, 22) elektrische Kontaktbereiche der Fadenanordnung definieren;
wobei der Mittelabschnitt (3) und die zwei Seitenabschnitte (2, 4, 20, 22) jeweils eine Vielzahl von Öffnungen aufweisen, wobei die Vielzahl von Öffnungen der Mittelfläche eine offene Fläche des Mittelabschnitts (3) definiert und die Vielzahl von Öffnungen jedes Seitenabschnitts (2, 4, 20, 22) eine offene Fläche der Seitenabschnitte (2, 4, 20, 22) definiert;
**dadurch gekennzeichnet, dass** der Prozentsatz der Gesamtfläche des Mittelabschnitts (3), der die offene Fläche des Mittelabschnitts (3) aufweist, größer ist als der Prozentsatz der Gesamtfläche eines der Seitenabschnitte (2, 4, 20, 22), der die offene Fläche des Seitenabschnitts (2, 4, 20, 22) aufweist.

2. Flache Fadenanordnung nach Anspruch 1, wobei ein Verhältnis des Prozentsatzes der Gesamtfläche des Mittelabschnitts (3), der die offene Fläche des Mittelabschnitts (3) aufweist, zu dem Prozentsatz der Gesamtfläche eines der beiden Seitenabschnitte (2, 4, 20, 22), der die offene Fläche des Seitenabschnitts (2, 4, 20, 22) aufweist, zwischen 1,1 und 30 beträgt.

3. Flache Fadenanordnung nach Anspruch 1 oder 2, wobei die offene Fläche des Mittelabschnitts (3) zwischen 40 Prozent und 90 Prozent der Gesamtfläche des Mittelabschnitts (3) beträgt;
und wobei die offene Fläche in jedem der beiden Seitenabschnitte (2, 4, 20, 22) größer als 3 Prozent und kleiner als 40 Prozent von jeder der Gesamtflächen der beiden Seitenabschnitte (2, 4, 20, 22) ist.

4. Flache Fadenanordnung nach einem der Ansprüche 1 bis 3, wobei ein Übergangsabschnitt (32) zwischen jedem der beiden Seitenabschnitte (2, 4, 20, 22) und dem Mittelabschnitt (3) angeordnet ist, wobei jeder Übergangsabschnitt (32) eine Vielzahl von Öffnungen aufweist, die eine offene Fläche definieren, und wobei die Verteilung der offenen Fläche über den Übergangsabschnitt (32) zwischen dem Seitenabschnitt (2, 4, 20, 22) und dem Mittelabschnitt (3) variiert.

5. Flache Fadenanordnung nach einem der Ansprüche 1 bis 4, aufweisend einen mittleren Längsbereich, der sich von einem der beiden Seitenabschnitte (2, 4, 20, 22) über den Mittelabschnitt (3) zu dem anderen der beiden Seitenabschnitte (2, 4, 20, 22) erstreckt, wobei der Prozentsatz der Gesamtfläche des Mittelabschnitts (3) innerhalb des mittleren Längsbereichs, der die offene Fläche aufweist, kleiner ist als der Prozentsatz der Gesamtfläche des Mittelabschnitts (3) außerhalb des mittleren Längsbereichs, der die offene Fläche aufweist.

6. Flache Fadenanordnung nach einem der Ansprüche 1 bis 5, wobei die Fadenanordnung eine Netzanordnung (1) ist, wobei ein Netz des Mittelabschnitts (3) und Netze des ersten und des zweiten Seitenabschnitts (2, 4, 20, 22) jeweils eine Netzdichte aufweisen, wobei die Netzdichte in dem Mittelabschnitt (3) niedriger ist als die Netzdichte in jedem der ersten und zweiten Seitenabschnitte (2, 4, 20, 22) .

7. Flache Fadenanordnung nach Anspruch 6, wobei ein Netzdichtegradient sich zwischen dem ersten Seitenabschnitt (2, 4, 20, 22) und dem Mittelabschnitt (3) sowie zwischen dem Mittelabschnitt (3) und dem zweiten Seitenabschnitt (2, 4, 20, 22) befindet.

8. Flache Fadenanordnung nach einem der Ansprüche 6 oder 7, wobei das Netz verflochten ist und in Webrichtung der Fadenanordnung eine konstante Anzahl von Fäden entlang der gesamten Länge der Fadenanordnung nebeneinander angeordnet sind.

9. Flache Fadenanordnung nach einem der Ansprüche 6 bis 8, aufweisend einen mittleren Längsbereich, der sich von einem der beiden Seitenabschnitte (2, 4, 20, 22) über den Mittelabschnitt (3) zu dem anderen der beiden Seitenabschnitte (2, 4, 20, 22) erstreckt, wobei das Netz verflochten ist und in Webrichtung der Fadenanordnung mehr Fäden im mittleren Längsbereich als außerhalb des mittleren Längsbereichs angeordnet sind.

10. Fluiddurchlässige Heizvorrichtungsbaugruppe für Aerosolerzeugungssysteme, wobei die fluiddurchlässige Heizvorrichtungsbaugruppe eine elektrisch leitfähige, flache Fadenanordnung nach einem der Ansprüche 1 bis 9 aufweist, und
einen ersten Kontaktpunkt (28, 48) und einen zweiten Kontaktpunkt (28, 48) zum elektrischen Kontaktieren der flachen Fadenanordnung, wobei eine Längsachse zwischen dem ersten Kontaktpunkt (28, 48) und dem zweiten Kontaktpunkt (28, 48) definiert ist,
wobei eine Mittelfläche Sc (10) ein Bereich der Heizvorrichtungsbaugruppe ist, der sich zwischen zwei Linien erstreckt, die senkrecht zur Längsachse liegen und die Längsachse an zwei Punkten kreuzen, die auf der Längsachse angeordnet sind, wobei einer der beiden Punkte sich in einem Abstand von dem ersten Kontaktpunkt (28, 48) befindet, der gleich 40 Prozent ist, und der andere der beiden Punkte sich in einem Abstand von dem ersten Kontaktpunkt (28, 48), befindet, der gleich 60 Prozent des Abstands zwischen dem ersten und dem zweiten Kontaktpunkt (28, 48) ist;
wobei eine erste Seitenfläche S1 (11) ein Bereich der Heizvorrichtungsbaugruppe ist, der sich zwischen zwei Linien erstreckt, die senkrecht zur Längsachse liegen und die Längsachse an dem ersten Kontaktpunkt (28, 48) kreuzen, und einem Punkt, der auf der Längsachse angeordnet ist und sich in einem Abstand von dem ersten Kontaktpunkt (28, 48) befindet, der gleich 20 Prozent des Abstands zwischen dem ersten und dem zweiten Kontaktpunkt (28, 48) ist;
wobei eine zweite Seitenfläche S2 (15) ein Bereich der Heizvorrichtungsbaugruppe zwischen zwei Linien ist, die senkrecht zur Längsachse liegen und die Längsachse an dem zweiten Kontaktpunkt (28, 48) kreuzen, und einem Punkt, der auf der Längsachse angeordnet ist und sich in einem Abstand von dem ersten Kontaktpunkt (28, 48) befindet, der gleich 80 Prozent des Abstands zwischen dem ersten und dem zweiten Kontaktpunkt (28, 48) ist;
wobei die Mittelfläche Sc (10) eine Vielzahl von Öffnungen aufweist, die einen offenen Bereich ScOA definieren, wobei die erste Seitenfläche S1 (11) eine Vielzahl von Öffnungen aufweist, die einen offenen Bereich S1OA definieren, und die zweite Seitenfläche S2 (15) eine Vielzahl von Öffnungen aufweist, die einen offenen Bereich S2OA definieren, und
wobei der Prozentsatz der Gesamtfläche der Mittelfläche (10), die die offene Fläche der Mittelfläche (10) aufweist, größer ist als der Prozentsatz der Gesamtfläche der ersten Seitenfläche (11), die die offene Fläche der ersten Seitenfläche (11) aufweist, und
wobei der Prozentsatz der Gesamtfläche der Mittelfläche (10), die die offene Fläche der Mittelfläche (10) aufweist, größer ist als der Prozentsatz der Gesamtfläche der zweiten Seitenfläche (15), die die offene Fläche der zweiten Seite aufweist.

11. Heizvorrichtungsbaugruppe nach Anspruch 10, wobei ein Verhältnis des Prozentsatzes der Gesamtfläche der Mittelfläche (10), die die offene Fläche der Mittelfläche (10) aufweist, zu dem Prozentsatz der Gesamtfläche der Mittelfläche (10), die die offene Fläche der ersten Seitenfläche (11) ScOA/S1OA aufweist, zwischen 1,1 und 30 beträgt, und wobei ein Verhältnis des Prozentsatzes der Gesamtfläche der Mittelfläche (10), die die offene Fläche der Mittelfläche (10) aufweist, zu dem Prozentsatz der Gesamtfläche der zweiten Seitenfläche (15), die die offene Fläche der zweiten Seitenfläche (15) ScOA/S2OA aufweist, zwischen 1,1 und 30 beträgt.

12. Heizvorrichtungsbaugruppe nach Anspruch 10 oder 11, wobei die offene Fläche der Mittelfläche (10) ScOA zwischen 40 Prozent und 90 Prozent der Gesamtfläche der Mittelfläche (10) beträgt;
und wobei die offene Fläche in jeder der beiden Seitenflächen (11, 15) S1OA, S2OA größer als 3 Prozent und kleiner als 40 Prozent jeder der Gesamtflächen der beiden Seitenflächen (11, 15) ist.

13. Heizvorrichtungsbaugruppe nach einem der Ansprüche 10 bis 12, ferner aufweisend eine erste Übergangsfläche (12), die ein Bereich der Heizvorrichtungsbaugruppe ist, der sich zwischen zwei Linien erstreckt, die senkrecht zur Längsachse liegen und die Längsachse an zwei Punkten kreuzen, die auf der Längsachse angeordnet sind, wobei einer der beiden Punkte sich in einem Abstand vom ersten Kontaktpunkt (28, 48) befindet, der gleich 20 Prozent des Abstands ist, und der andere der beiden Punkte sich in einem Abstand von dem ersten Kontaktpunkt (28, 48) befindet, der gleich 40 Prozent des Abstands zwischen dem ersten und dem zweiten Kontaktpunkt (28, 48) ist; und
wobei eine zweite Übergangsfläche (14) ein Bereich der Heizvorrichtungsbaugruppe ist, der sich zwischen zwei Linien erstreckt, die senkrecht zur Längsachse liegen und die Längsachse an zwei Punkten kreuzen, die auf der Längsachse angeordnet sind, wobei einer der beiden Punkte sich in einem Abstand von dem ersten Kontaktpunkt (28, 48) befindet, der gleich 60 Prozent ist, und der andere der beiden Punkte sich in einem Abstand von dem ersten Kontaktpunkt (28, 48) befindet, der gleich 80 Prozent des Abstands zwischen dem ersten und dem zweiten Kontaktpunkt (28, 48) ist,
wobei die erste Übergangsfläche (12) eine Vielzahl von Öffnungen aufweist, die einen offenen Bereich T1OA definieren, und die zweite Übergangsfläche (14) eine Vielzahl von Öffnungen aufweist, die einen offenen Bereich T2OA definieren,
wobei ein Verhältnis des Prozentsatzes der Gesamtfläche der ersten Übergangsfläche (12), die die offene Fläche der ersten Übergangsfläche (12) aufweist, zu dem Prozentsatz der Gesamtfläche der ersten Seitenfläche (11), die die offene Fläche der ersten Seitenfläche (11) T1OA/S1OA aufweist, zwischen 1 und 30 beträgt,
wobei ein Verhältnis des Prozentsatzes der Gesamtfläche der zweiten Übergangsfläche (14), die die offene Fläche der zweiten Übergangsfläche (14) aufweist, zu dem Prozentsatz der Gesamtfläche der zweiten Seitenfläche (15), die die offene Fläche der zweiten Seitenfläche (15) T2OA/S2OA aufweist, zwischen 1 und 30 beträgt.

14. Heizvorrichtungsbaugruppe nach einem der Ansprüche 10 bis 13, wobei ein Mittelwiderstand Rc der elektrische Widerstand der Mittelfläche (10) entlang der Längsachse ist, wobei ein erster Widerstand R1 ein elektrischer Widerstand der ersten Seitenfläche (11) entlang der Längsachse ist, wobei ein zweiter Widerstand R2 ein elektrischer Widerstand der zweiten Seitenfläche (15) entlang der Längsachse ist;
und wobei ein Verhältnis des Mittelwiderstands zum ersten Widerstand Rc/R1 zwischen 2 und 400 beträgt und wobei ein Verhältnis des Mittelwiderstands zum zweiten Widerstand Rc/R2 zwischen 2 und 400 beträgt.

15. Heizvorrichtungsbaugruppe nach einem der Ansprüche 10 bis 14, wobei ein Gesamtwiderstand Rt, der dem elektrischen Widerstand zwischen der ersten Kontaktstelle (28, 48) und der zweiten Kontaktstelle (28, 48) entspricht, zwischen 0,5 Ohm und 4 Ohm ist, wobei der Mittelwiderstand Rc höher als 0,5 Ohm ist, wobei der erste Widerstand R1 und der zweite Widerstand R2 jeweils niedriger als 100 mOhm sind.

16. Heizvorrichtungsbaugruppe nach einem der Ansprüche 10 bis 15, aufweisend ein Substrat, das eine Öffnung durch das Substrat aufweist;
wobei sich die elektrisch leitfähige, flache Fadenanordnung über die Öffnung in dem Substrat erstreckt; und
ein Befestigungselement, das die flache Fadenanordnung an dem Substrat befestigt.

17. Elektrisch betriebenes Aerosolerzeugungssystem, aufweisend:
eine Aerosolerzeugungsvorrichtung und eine Patrone, die ein flüssiges aerosolbildendes Substrat aufweist;
eine fluiddurchlässige Heizvorrichtungsbaugruppe nach einem der Ansprüche 10 bis 16 oder
eine fluiddurchlässige Heizvorrichtungsbaugruppe, die eine flache Fadenanordnung nach einem der Ansprüche 1 bis 9 aufweist,
wobei die Patrone ein Gehäuse mit einer Öffnung aufweist, wobei sich die Heizvorrichtungsbaugruppe über die Öffnung des Gehäuses der Patrone erstreckt,
und wobei die Aerosolerzeugungsvorrichtung einen Hauptkörper aufweist, der einen Hohlraum zum Aufnehmen der Patrone, eine elektrische Energiequelle und elektrische Kontakte zum Verbinden der elektrischen Energiequelle mit der Heizvorrichtungsbaugruppe definiert.

## Revendications

1. Agencement de filaments plats électriquement conducteur pour un ensemble chauffant perméable aux fluides pour des systèmes de génération d'aérosol, l'agencement de filaments plats comprenant :
une partie centrale (3) et deux parties latérales (2, 4, 20, 22), les deux parties latérales (2, 4, 20, 22) étant agencées sur des côtés opposés de la partie centrale (3), dans lequel la partie centrale (3) définit une région chauffante de l'agencement de filaments et les parties latérales (2, 4, 20, 22) définissent des régions de contact électrique de l'agencement de filaments ;
la partie centrale (3) et les deux parties latérales (2, 4, 20, 22) comprennent chacune une pluralité d'ouvertures, la pluralité d'ouvertures de l'aire centrale définissant une aire ouverte de la partie centrale (3) et la pluralité d'ouvertures de chaque partie latérale (2, 4, 20, 22) définissant une aire ouverte des parties latérales (2, 4, 20, 22) ;
**caractérisé en ce que** le pourcentage de l'aire totale de la partie centrale (3) comprenant l'aire ouverte de la partie centrale (3) est supérieur au pourcentage de l'aire totale de l'une des parties latérales (2, 4, 20, 22) comprenant l'aire ouverte de la partie latérale (2, 4, 20, 22).

2. Agencement de filaments plats selon la revendication 1, dans lequel un rapport du pourcentage de l'aire totale de la partie centrale (3) comprenant l'aire ouverte de la partie centrale (3) sur le pourcentage de l'aire totale de l'une des deux parties latérales (2, 4, 20, 22) comprenant l'aire ouverte de la partie latérale (2, 4, 20, 22) est entre 1,1 et 30.

3. Agencement de filaments plats selon la revendication 1 ou 2, dans lequel l'aire ouverte de la partie centrale (3) est entre 40 pour cent et 90 pour cent de l'aire totale de la partie centrale (3) ;
dans lequel l'aire ouverte dans chacune des deux parties latérales (2, 4, 20, 22) est supérieure à 3 pour cent et inférieure à 40 pour cent de chacune des aires totales des deux parties latérales (2, 4, 20, 22).

4. Agencement de filaments plats selon l'une quelconque des revendications 1 à 3, dans lequel une partie de transition (32) est agencée entre chacune des deux parties latérales (2, 4, 20, 22) et la partie centrale (3), chaque partie de transition (32) comprenant une pluralité d'ouvertures définissant une aire ouverte, et dans lequel la distribution de l'aire ouverte à travers la partie de transition (32) varie entre la partie latérale (2, 4, 20, 22) et la partie centrale (3).

5. Agencement de filaments plats selon l'une quelconque des revendications 1 à 4, comprenant une région longitudinale centrale s'étendant depuis l'une des deux parties latérales (2, 4, 20, 22) sur la partie centrale (3) vers l'autre des deux parties latérales (2, 4, 20, 22), dans lequel le pourcentage de l'aire totale de la partie centrale (3) à l'intérieur de la région longitudinale centrale comprenant une aire ouverte est inférieur au pourcentage de l'aire totale de la partie centrale (3) à l'extérieur de la région longitudinale centrale comprenant une aire ouverte.

6. Agencement de filaments plats selon l'une quelconque des revendications 1 à 5, dans lequel l'agencement de filaments est un agencement de treillis (1), dans lequel un treillis de la partie centrale (3) et des treillis des première et seconde parties latérales (2, 4, 20, 22) comprennent chacun une densité de treillis, dans lequel la densité de treillis dans la partie centrale (3) est inférieure à la densité de treillis dans chacune des première et seconde parties latérales (2, 4, 20, 22).

7. Agencement de filaments plats selon la revendication 6, dans lequel un gradient de densité de treillis est situé entre la première partie latérale (2, 4, 20, 22) et la partie centrale (3) et entre la partie centrale (3) et la seconde partie latérale (2, 4, 20, 22).

8. Agencement de filaments plats selon l'une des revendications 6 ou 7, dans lequel le treillis est tissé et, dans une direction de tissage de l'agencement de filaments, un nombre constant de filaments sont agencés les uns à côté des autres sur toute la longueur de l'agencement de filaments.

9. Agencement de filaments plats selon l'une quelconque des revendications 6 à 8, comprenant une région longitudinale centrale s'étendant depuis l'une des deux parties latérales (2, 4, 20, 22) sur la partie centrale (3) jusqu'à l'autre des deux parties latérales (2, 4, 20, 22), dans lequel le treillis est tissé et, dans la direction de tissage de l'agencement de filaments, plus de filaments sont agencés dans la région longitudinale centrale qu'à l'extérieur de la région longitudinale centrale.

10. Ensemble chauffant perméable aux fluides pour des systèmes de génération d'aérosols, l'ensemble chauffant perméable aux fluides comprenant un agencement de filaments plats électriquement conducteur selon l'une quelconque des revendications 1 à 9, et
un premier point de contact (28, 48) et un second point de contact (28, 48) pour une mise en contact électrique avec l'agencement de filaments plats, dans lequel un axe longitudinal est défini entre le premier point de contact (28, 48) et le second point de contact (28, 48),
dans lequel une surface centrale Sc (10) est une aire de l'ensemble de chauffage s'étendant entre deux lignes reposant perpendiculairement à l'axe longitudinal et croisant l'axe longitudinal au niveau de deux points agencés sur l'axe longitudinal, un des deux points étant situé à une distance du premier point de contact (28, 48) égale à 40 pour cent et l'autre des deux points étant situé à une distance du premier point de contact (28, 48) égale à 60 pour cent de la distance entre le premier et le second point de contact (28, 48) ;
dans lequel une première surface latérale S1 (11) est une aire de l'ensemble de chauffage s'étendant entre deux lignes reposant perpendiculairement à l'axe longitudinal et croisant l'axe longitudinal au niveau du premier point de contact (28, 48) et un point agencé sur l'axe longitudinal et situé à une distance du premier point de contact (28, 48) égale à 20 pour cent de la distance entre le premier et le second point de contact (28, 48) ;
dans lequel une seconde surface latérale S2 (15) est une aire de l'ensemble de chauffage entre deux lignes reposant perpendiculairement à l'axe longitudinal et croisant l'axe longitudinal au niveau du second point de contact (28, 48) et un point agencé sur l'axe longitudinal et situé à une distance du premier point de contact (28, 48) égale à 80 pour cent de la distance entre le premier et le second point de contact (28, 48) ;
dans lequel la surface centrale Sc (10) comprend une pluralité d'ouvertures définissant une aire ouverte ScAO, la première surface latérale S1 (11) comprend une pluralité d'ouvertures définissant une aire ouverte S1AO, et la seconde surface latérale S2 (15) comprend une pluralité d'ouvertures définissant une aire ouverte S2AO, et
dans lequel le pourcentage de l'aire totale de la surface centrale (10) comprenant l'aire ouverte de la surface centrale (10) est supérieur au pourcentage de l'aire totale de la première surface latérale (11) comprenant l'aire ouverte de la première surface latérale (11) et
dans lequel le pourcentage de l'aire totale de la surface centrale (10) comprenant l'aire ouverte de la surface centrale (10) est supérieur au pourcentage de l'aire totale de la seconde surface latérale (15) comprenant l'aire ouverte du second côté.

11. Ensemble de chauffage selon la revendication 10, dans lequel un rapport du pourcentage de l'aire totale de la surface centrale (10) comprenant l'aire ouverte de la surface centrale (10) sur le pourcentage de l'aire totale de la surface centrale (10) comprenant l'aire ouverte de la première surface latérale (11) ScAO/S1AO est entre 1,1 et 30, et dans lequel un rapport du pourcentage de l'aire totale de la surface centrale (10) comprenant l'aire ouverte de la surface centrale (10) sur le pourcentage de l'aire totale de la seconde surface latérale (15) comprenant l'aire ouverte de la seconde surface latérale (15) ScAO/S2AO est entre 1,1 et 30.

12. Ensemble de chauffage selon les revendications 10 ou 11, dans lequel l'aire ouverte de la surface centrale (10) ScAO est entre 40 pour cent et 90 pour cent de l'aire totale de la surface centrale (10) ;
et dans lequel l'aire ouverte dans chacune des deux surfaces latérales (11, 15) S1AO, S2AO est supérieure à 3 pour cent et inférieure à 40 pour cent de chacune des aires totales des deux surfaces latérales (11, 15).

13. Ensemble de chauffage selon l'une quelconque des revendications 10 à 12, comprenant en outre une première surface de transition (12) étant une surface de l'ensemble de chauffage s'étendant entre deux lignes reposant perpendiculairement à l'axe longitudinal et croisant l'axe longitudinal au niveau de deux points agencés sur l'axe longitudinal, un des deux points étant situé à une distance du premier point de contact (28, 48) égale à 20 pour cent et l'autre des deux points étant situé à une distance du premier point de contact (28, 48) égale à 40 pour cent de la distance entre le premier et le second point de contact (28, 48) ; et
une seconde surface de transition (14) étant une aire de l'ensemble de chauffage s'étendant entre deux lignes reposant perpendiculairement à l'axe longitudinal et croisant l'axe longitudinal au niveau de deux points agencés sur l'axe longitudinal, un des deux points étant situé à une distance du premier point de contact (28, 48) égale à 60 pour cent et l'autre des deux points étant situé à une distance du premier point de contact (28, 48) égale à 80 pour cent de la distance entre le premier et le second point de contact (28, 48),
dans lequel la première surface de transition (12) comprend une pluralité d'ouvertures définissant une aire ouverte T1AO, et la seconde surface de transition (14) comprend une pluralité d'ouvertures définissant une aire ouverte T2AO,
dans lequel un rapport du pourcentage de l'aire totale de la première surface de transition (12) comprenant l'aire ouverte de la première surface de transition (12) sur le pourcentage de l'aire totale de la première surface latérale (11) comprenant l'aire ouverte de la première surface latérale (11) T1AO/S1AO est entre 1 et 30,
dans lequel un rapport du pourcentage de l'aire totale de la seconde surface de transition (14) comprenant l'aire ouverte de la seconde surface de transition (14) sur le pourcentage de l'aire totale de la seconde surface latérale (15) comprenant l'aire ouverte de la seconde surface latérale (15) T2AO/S2AO est entre 1 et 30.

14. Ensemble de chauffage selon l'une quelconque des revendications 10 à 13, dans lequel une résistance centrale Rc est la résistance électrique de la surface centrale (10) le long de l'axe longitudinal, dans lequel une première résistance R1 est une résistance électrique de la première surface latérale (11) le long de l'axe longitudinal, dans lequel une seconde résistance R2 est une résistance électrique de la seconde surface latérale (15) le long de l'axe longitudinal ;
dans lequel un rapport de la résistance centrale sur la première résistance R1 est entre 2 et 400, et dans lequel un rapport de la résistance centrale sur la seconde résistance Rc/R2 est entre 2 et 400.

15. Ensemble de chauffage selon l'une quelconque des revendications 10 à 14, dans lequel une résistance totale Rt correspondant à la résistance électrique entre le premier point de contact (28, 48) et le second point de contact (28, 48) est entre 0,5 Ohm et 4 Ohms, dans lequel la résistance centrale Rc est supérieure à 0,5 Ohm, dans lequel la première résistance R1 et la seconde résistance R2 sont chacune inférieures à 100 mOhm.

16. Ensemble de chauffage selon l'une quelconque des revendications 10 à 15, comprenant un substrat comprenant une ouverture à travers le substrat ;
dans lequel l'agencement de filaments plats électriquement conducteur s'étend sur l'ouverture dans le substrat ; et une attache fixant l'agencement de filaments plats au substrat.

17. Système de génération d'aérosol à fonctionnement électrique comprenant :
un dispositif de génération d'aérosol et une cartouche comprenant un substrat formant aérosol liquide ;
un ensemble chauffant perméable aux fluides selon l'une quelconque des revendications 10 à 16 ou
un ensemble chauffant perméable aux fluides comprenant un agencement de filaments plats selon l'une quelconque des revendications 1 à 9,
dans lequel la cartouche comprend un logement ayant une ouverture, avec l'ensemble de chauffage s'étendant à travers l'ouverture du logement de la cartouche,
et dans lequel le dispositif de génération d'aérosol comprend un corps principal définissant une cavité pour recevoir la cartouche, une source d'énergie électrique, et des contacts électriques pour raccorder la source d'énergie électrique à l'ensemble de chauffage.
